# EUROPEAN PATENT APPLICATION

(11) **EP 2 431 085 A1**
(43) Date of publication of application: **21.03.2012**
(21) Application number: 11382223.3
(22) Date of filing: 04.07.2011
(51) Int. Cl.: B01D 17/06, C07C 29/76, C07C 29/86

(54) **Process for purifying glycerin derived from biodiesel production**

(30) Priority: 20.09.2010 ES 201031399
(71) Applicant: FUNDACION TECNALIA RESEARCH & INNOVATION, 48160 Derio Vizcaya (ES)
(72) Inventor: Belaustegi Ituarte, Yolanda, 48160 Derio (Vizcaya) (ES); Lorenzo Ibarreta, Leire, 48160 Derio (Vizcaya) (ES); Torrecilla Soria, Jesús, 48160 Derio (Vizcaya) (ES); Gomez Jiménez de Aberasturi, Olga, 48160 Derio (Vizcaya) (ES); Marquinez Porres, Rosa, 48160 Derio (Vizcaya) (ES)
(74) Representative: Carpintero Lopez, Francisco

(57) **Abstract**

A process for purifying glycerin derived from biodiesel production is described, the process comprises acidifying the glycerin; neutralizing the acidified glycerin; feeding the glycerin into an electrodialysis unit comprising at least one cation exchange membrane and at least one anion exchange membrane separated between an anode and a cathode, such that they define at least one diluted compartment and at least one concentrated compartment located between the anode and the cathode, wherein the glycerin is fed into the diluted compartment and an NaCl solution is fed into the concentrated compartment; applying a potential difference so that the ions present in the glycerin migrate through the anion and cation exchange membranes from the diluted compartments to the concentrated compartments, where they are trapped, purifying the glycerin; and extracting the purified glycerin.

## Description

### Field of the Invention

The present invention relates to the techniques used in the chemical industry for purifying substances, and it more particularly relates to a process for purifying glycerin produced as a byproduct in the biodiesel manufacture.

### Background of the Invention

Glycerin has the potential of being an extremely versatile "building block" in biorefining. However, crude glycerin derived from biodiesel production has a very low quality due to the presence of impurities, their concentration percentage ranging between approximately 50 and 80%. The substances accompanying glycerin are mainly water, methanol, inorganic salts, fatty acids, unreacted triglycerides and other matter in variable amounts.

The decision of how to subsequently refine crude glycerin depends on the economies of the production scale and/or on the availability of purification facilities.

Major biodiesel producers usually refine the glycerin produced and sell it for other industries. Generally, the operations used for purifying glycerin are: filtration, addition of chemicals and vacuum fractional distillation, producing various commercial qualities or grades.

However, if the glycerin is to be used in food, cosmetics and pharmacy, a higher degree of purification is required, including phases of bleaching, deodorizing and ion exchange for removing trace impurities and salts.

The purification to that grade is expensive and generally not within the economic capacity of small- and medium-scale plants. As a result, the glycerin is often incinerated, which can give rise to toxic acrolein emissions.

### Summary of the Invention

To overcome the problems of the prior art, a new process is provided for purifying glycerin by means of an economically viable treatment adaptable to small-and medium-sized enterprises for purifying the glycerin, particularly the process of the present invention is based on membrane technology, and specifically, electrodialysis.

More particularly, the process of the present invention comprises:
a) acidifying the glycerin;
b) treating the acidified glycerin with a base;
c) feeding the glycerin into an electrodialysis unit comprising at least one cation exchange membrane and at least one anion exchange membrane separated between an anode and a cathode, such that they define at least one diluted compartment and at least one concentrated compartment located between the anode and the cathode, wherein the glycerin is fed into the diluted compartment and an NaCl solution is fed into the concentrated compartment;
d) applying a potential difference between the anode and the cathode so that the ions present in the glycerin migrate through the anion and cation exchange membranes from the diluted compartments to the concentrated compartments, where they are trapped, purifying the glycerin; and
e) extracting the purified glycerin from the electrodialysis unit.

In an embodiment of the invention, after the phase of neutralization with the base, the glycerin is diluted with water up to 50% to improve electrodialysis. In yet another embodiment, the glycerin that is extracted from the electrodialysis unit is clarified with an adsorber. In another additional embodiment, the water is removed from the treated glycerin, preferably by means of evaporation.

The process of the present invention allows purifying glycerin in a practical and cost-effective manner and in a continuous manner.

### Brief Description of the Drawings

To complement the description that is being made and for the purpose of aiding to better understand the features of the invention according to a preferred practical embodiment thereof, a set of drawings is attached as an integral part of this description in which the following is depicted with an illustrative and non-limiting character:
Figure 1 is a schematic depiction of an example of an electrodialysis unit where the main phase of the process of the present invention is performed.
Figure 2 shows an infrared spectrum of the glycerin produced by means of the process of the invention.
Figure 3 shows a reference infrared spectrum with glycerin 85%.

### Detailed Description of the Preferred Embodiments of the Invention

The process of the present invention starts with acidifying the glycerin with an inorganic acid, the acidification allows removing soaps, fats, etc., from the glycerin to be treated, hydrochloric acid is preferably used for this phase, although sulfuric acid, phosphoric acid or another acid can also be used. The acidification is performed up to a pH of 4 to 5. The acid concentration used is usually 2-3 mol/l. In the case of HCl, since it has a commercial grade purity of 36%, a concentrated form is normally used.

The acidified glycerin is subsequently treated with a base such as sodium hydroxide, sodium borohydride, calcium hydroxide, potassium hydroxide, mixtures thereof and other similar bases. The treatment with the base is performed until reaching pH=6 and is carried out with approximately 15% of the base used.

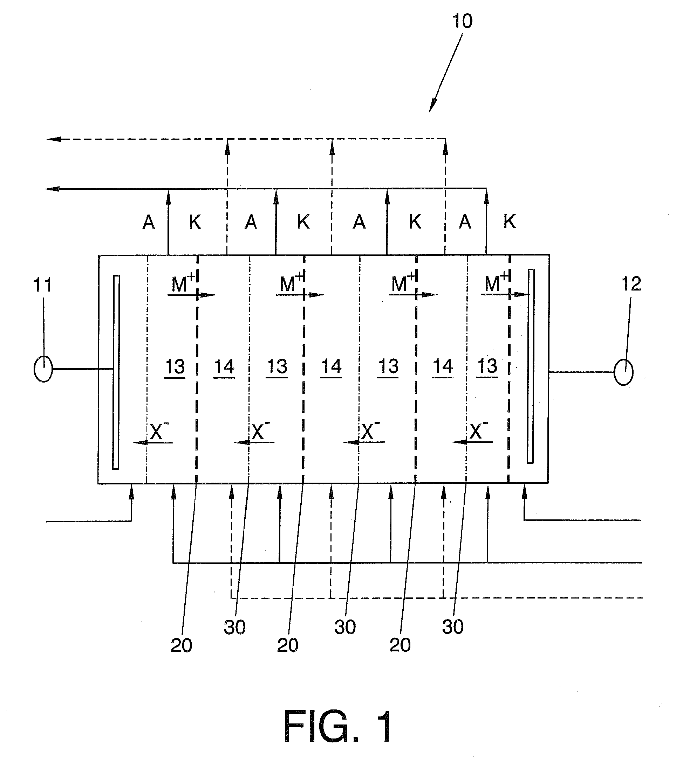

Once the glycerin has been treated with the base, electrolytic treatment is performed in an electrodialysis unit 10, such as the one depicted in Figure 1; electrodialysis is a membrane technology which, under the influence of a continuous electric field allows extracting ionized organic and inorganic substances dissolved in aqueous solution through selective ion exchange membranes. In an embodiment of the invention, the glycerin is diluted using up to 50% water before subjecting it to electrodialysis.

More particularly, it can be seen in Figure 1 that the electrodialysis unit 10 comprises at least one cation exchange membrane 20 (permeable only to cations) and at least one anion exchange membrane 30 (permeable only to anions) arranged in an alternating manner between an anode 11 and a cathode 12, such that they give rise to at least one diluted compartment 13 and at least one concentrated compartment 14.

The glycerin solution, the ions of which are to be extracted, enters and circulates in each diluted compartment 13, and an NaCl solution with a concentration of up to 30 g/l, preferably 10 g/l, in which said ions are going to be concentrated, enters and circulates in each concentrated compartment 14. When applying a potential difference between the anode and the cathode 11 and 12, the anions X⁻ migrate through the anion exchange membranes 30, from the compartments 13 to the compartments 14, where they are trapped, because the ion barrier formed by any one of the cation exchange membranes 20 are placed in their path.

Analogously, the cations M⁺ migrate, through the cation exchange membranes 20, from the compartments 13 to the compartments 14, where they are concentrated since the anionic membranes prevent their migration towards the cathode. The electrode reactions are only used for the purpose of providing the electric field necessary for the process to take place.

In the electrodialysis unit, the anionic membrane can be a Neosepta AMX, Neosepta AFN or AFX type membrane, whereas the cationic membrane is Neosepta CMX or others that are well known in the art. In order to make this phase of the process more efficient, the applied current density is 100 to 500 A/m².

After electrodialysis, if necessary, the glycerin is subjected to a step of clarification, for this an adsorbent agent, such as activated charcoal, clays and ion exchange resins.

Likewise, in order to concentrate the glycerin, this one is subjected to a step of removing the water, which can be done by evaporation.

The process of the present invention will be more clearly illustrated by means of the examples described below which are presented for merely illustrative and non-limiting purposes, said examples being the followings

### Examples

The process of the present invention was put into practice for purifying glycerin produced in a biodiesel manufacturing process, the feed current of the glycerin to be purified had a concentration of approximately 50%, including among its impurities unconverted triglycerides, methanol, soaps, etc., it furthermore had a dark brown color.

Before the electrodialysis phase, the glycerin produced as a byproduct in biodiesel production was treated with HCl for the purpose of producing a soap and fat-free glycerin, and then it was treated with sodium hydroxide to remove the excess acid, obtaining a brown colored glycerin solution contaminated with salts, water and methanol, having the properties indicated in Table 1.

**Table 1**

| Properties of glycerin | |
|---|---|
| pH | 5.7 |
| % Glycerin | 85.8 |
| Conductivity | 294 µS/cm |
| Density | 1.2949 g/mL |
| [Cl⁻] | 60.41 g/L |
| Color | Brown |

Subsequently, the conditioned glycerin was subjected to electrodialysis in a two-compartment press filter-type laboratory cell provided with 2 electrodes, an anion exchange membrane and a cation exchange membrane with a configuration similar to the one shown in Figure 1.

The anode used was an IrO₂/Ti (DSA-O₂) anode and the cathode was a 316 stainless steel cathode. Neosepta AMX membranes and Neosepta AFN and AFX diffusion membranes have been used as anionic membranes, and Neosepta CMX membranes have been used as cationic membranes.

The distance between membranes was set at 3.3 cm and the effective membrane area was 20 cm².

The different solutions, contained in glass tanks, were recirculated through their corresponding compartments by means of IWAKI MD-20R magnetic displacement pumps.

The electric current was applied by means of a 5A-60V direct current power supply. The glycerin solution from which the chloride ions are to be extracted was fed through the diluted compartment and a diluted sodium chloride solution where the chloride ions are concentrated was fed through the concentrated compartment.

The anodic reaction was the oxidation of water to oxygen with the simultaneous production of H⁺ ions and the cathodic reaction was the reduction of water to hydrogen, producing OH⁻ ions. Under the action of the electric field, the salts are extracted from the feed solution.

Electrodialysis was carried out with real samples and by determining the influence of the different variables on the process efficacy and the chloride concentration in the glycerin solution.

The process efficacy was monitored by analyzing the variation of the chloride concentration in the glycerin solution produced; in particular, the variables studied were: glycerin solution dilution percentage, anionic membrane and current density on the process efficacy, which will be discussed below.

### Examples 1- 3

Glycerin dilution percentage: The crude glycerin solution used was a very dense solution with barely any conductivity which had to be diluted with water in order to apply the treatment for removing chlorides by electrodialysis.

To that end, solutions with different glycerin/water percentages which are indicated in Table 2, were prepared. In Table 2 it can be observed that the conductivity increases with the dilution percentage, as well as the density decreases.

**Table 2**

| Properties of the glycerin/water solutions | | | | |
|---|---|---|---|---|
| %Glycerin | %Water | Density (g/ml) | pH | Conductivity (mS/cm) |
| 50 | 50 | 1.1356 | 6.36 | 18.08 |
| 60 | 40 | 1.1700 | 6.48 | 12.86 |
| 70 | 30 | 1.2020 | 6.52 | 8.76 |
| 80 | 20 | 1.2400 | 6.03 | 4.31 |
| 100 | 0 | 1.2949 | 5.70 | 0.25 |

Table 3 shows the results from the experiments conducted with glycerin solutions having different dilutions at a current density of 500 A/m² and working with the AMX anionic membrane and the CMX cationic membrane.

**Table 3**

| Results varying the glycerin dilution percentage | | | | | | | | | |
|---|---|---|---|---|---|---|---|---|---|
| Ex. No. | [Glyc] | T | Q | V | CD | [Cl⁻] | %Ec_{Cl}⁻ | %R_{Cl}⁻ | SEC |
| | 47.43 | 0 | 0 | 12.2 | 500 | 26.51 | --- | --- | --- |
| Ex. 1 | 46.68 | 1.60 | 0.060 | 17.24 | | 4.37 | 106.55 | 85.16 | 0.285 |
| | 51.67 | 0 | 0 | 12.60 | 500 | 29.86 | --- | --- | --- |
| Ex. 2 | 52.80 | 3.70 | 0.138 | 24.72 | | 1.53 | 58.00 | 95.18 | 0.821 |
| | 62.01 | 0 | 0 | 22.00 | 500 | 34.77 | --- | --- | --- |
| Ex. 3 | 65.70 | 3.58 | 0.134 | 26.12 | | 5.68 | 62.74 | 85.62 | 0.911 |

In Table 3: Effective membrane area: 20 cm²; distance between membranes: 3.3 cm; t: time (h); Q: circulated charge (Faradays); [Cl⁻]: chloride concentration (g/l) in the diluted compartment; CD: Current density (A/m²); V: voltage (volts); [Glyc]: % glycerin in the diluted compartment; Ec: current efficacy (%); R: reaction yield (%); SEC: Specific energy consumption (kWh/kg treated glycerin)

These results show that under the studied conditions, the smaller the dilution percentage the greater the energy consumption of the reaction.

It has also been seen that the glycerin is not diffused from the diluted compartment towards the concentrated compartment.

### Examples 4 and 5

### Current density

For the purpose of reducing the energy consumption different experiments were performed by reducing the current density and keeping the glycerin dilution percentage constant. The AMX anionic membrane and the CMX cationic membrane were used. Table 4 includes the results.

The results show that for a single dilution of 69%, as the working current density decreases the chloride removal percentage is high and close to 90%. However, together with the passage of chloride ions from the diluted solution to the concentrated solution, there is a glycerin diffusion of 13%.

**Table 4**

| Current density | | | | | | | | | |
|---|---|---|---|---|---|---|---|---|---|
| Ex. no. | [Glyc] | T | Q | V | CD | [Cl⁻] | %Ec_{Cl}⁻ | %R_{Cl}⁻ | SEC |
| Ex. 4 | 69.39 | 0 | 0 | 29.30 | 240 | 46.75 | --- | --- | --- |
| | 68.48 | 4.28 | 0.077 | 29.50 | | 20.49 | 101.71 | 59.24 | 0.833 |
| Ex. 5 | 68.84 | 0 | 0 | 10.53 | 150 | 41.25 | --- | --- | --- |
| | 56.36 | 12.28 | 0.137 | 26.40 | | 2.42 | 78.92 | 93.37 | 0.840 |

The abbreviations in Table 4 have the same meaning as those in Table 3

### Examples 6 and 7

### Anionic membrane

For the purpose of improving the process energy consumption Neosepta AFX and AFN anionic diffusion membranes were used for a single dilution percentage. The cationic membrane was Neosepta CMX and the working current density was 500 A/m².

The results (Table 5) showed that the anionic AFN membrane allows removing 99% of the chlorides from the sample with an efficacy of 72.14% and a specific energy consumption of 0.663 kWh/kg of treated glycerin.

This consumption was less if it is compared with that which is achieved when working with the AMX membrane. The chloride removal percentage is also high when working with the AFX membrane, however the current efficacy of the reaction decreases and the specific energy consumption increases.

A passage of glycerin ranging between approximately 6-7% from the diluted solution to the concentrated solution was also observed when using AFX and AFN membranes.

**Table 5**

| Anionic membrane, Cationic membrane: CMX, CD=500 A/m² | | | | | | | | | |
|---|---|---|---|---|---|---|---|---|---|
| Ex. no. | [Glyc] | T | Q | V | CD | [Cl⁻] | %Ec_{Cl}⁻ | %R_{Cl}⁻ | SEC |
| Ex. 6 | 60.08 | 0 | 0 | 13.00 | 500 | 34.75 | --- | --- | --- |
| (AFX) | 56.72 | 3.83 | 0.143 | 19.48 | | 4.08 | 60.88 | 88.93 | 0.700 |
| Ex. 7 | 60.08 | 0 | 0 | 11.80 | 500 | 34.75 | --- | --- | --- |
| (AFN) | 55.84 | 3.60 | 0.134 | 21.91 | | 0.31 | 72.14 | 99.00 | 0.663 |

The abbreviations have the same meaning as in Table 3

### Examples 8-10

### Anionic membrane AFN and lower current density

New experiments were conducted with the AFN membrane and by decreasing the current density. The studied current densities were 350 A/m², 250 A/m² and 150 A/m². The results are shown in Table 6.

These results showed that as the current density decreases, the energy consumption is also reduced and the process current efficacy increases, being the elimination of chlorides close to 100% in all cases.

In this case, glycerin diffusion from the diluted solution to the concentrated solution was also observed, being greater at lower current densities. This is because upon decreasing the current density, the time necessary for achieving a desalination grade increases and gives rise to greater glycerin losses due to diffusion, this process being dependent on the glycerin concentration in the diluted compartment, on the type of membrane and on the operating time.

Considering the global information resulting from these experiments, it is concluded that it is possible to remove virtually 100% of the chlorides present in the glycerin solution once it has been diluted with a current efficacy of 94% and an energy consumption of 0.36 kWh/kg of treated glycerin. This is possible by working with the AFN anionic membrane, the CMX cationic membrane and a current density of 150 A/m².

**Table 6**

| Ex. no. | [Glyc] | T | Q | V | CD | [Cl⁻] | %Ec_{Cl}⁻ | %R_{Cl}⁻ | SEC |
|---|---|---|---|---|---|---|---|---|---|
| Ex. 8 | 53.84 | 0 | 0 | 9.3 | 350 | 29.21 | --- | --- | --- |
| | 49.81 | 4.00 | 0.10 4 | 17.42 | | 0.39 | 77.72 | 98.66 | 0.422 |
| Ex. 9 | 53.77 | 0 | 0 | 8.2 | 250 | 32.50 | --- | --- | --- |
| | 45.87 | 6.00 | 0.11 2 | 17.91 | | 0.14 | 81.45 | 99.57 | 0.462 |
| Ex. 10 | 53.77 | 0 | 0 | 7.0 | 150 | 32.52 | --- | --- | --- |
| | 43.33 | 8.78 | 0.09 8 | 16.26 | | 0.06 | 94.01 | 99.81 | 0.362 |

The abbreviations have the same meaning as in Table 3

The removal of the chloride concentration from the glycerin solution is virtually 100%. However, it had a dark brown color, so the glycerin solution from the electrodialysis unit was treated with mineral coal powder, obtaining a colorless transparent solution.

In the event that this glycerin were to be used for pharmaceutical purposes, the chloride concentration therein must be less than or equal to 10 ppm. To meet this objective, the solution produced was treated with anion exchange resins in OH⁻ form, obtaining glycerin with a chloride concentration less than that.

Finally, once the colorless and chloride-free glycerin solution was produced, it was evaporated to remove the water, obtaining a glycerin 98% pure.

Table 7 summarizes the main properties of the pure glycerin and the glycerin produced by means of the process of the present invention.

**Table 7**

| Properties of the pure glycerin and the glycerin of this work | | |
|---|---|---|
| | Pure glycerin | Glycerin produced |
| Purity (%) | 99.4 | 97.9 |
| [Cl⁻] (ppm) | ---- | < 10 |
| Density (g/ml) | 1.259 | 1.259 |
| Color | Colorless | Colorless |

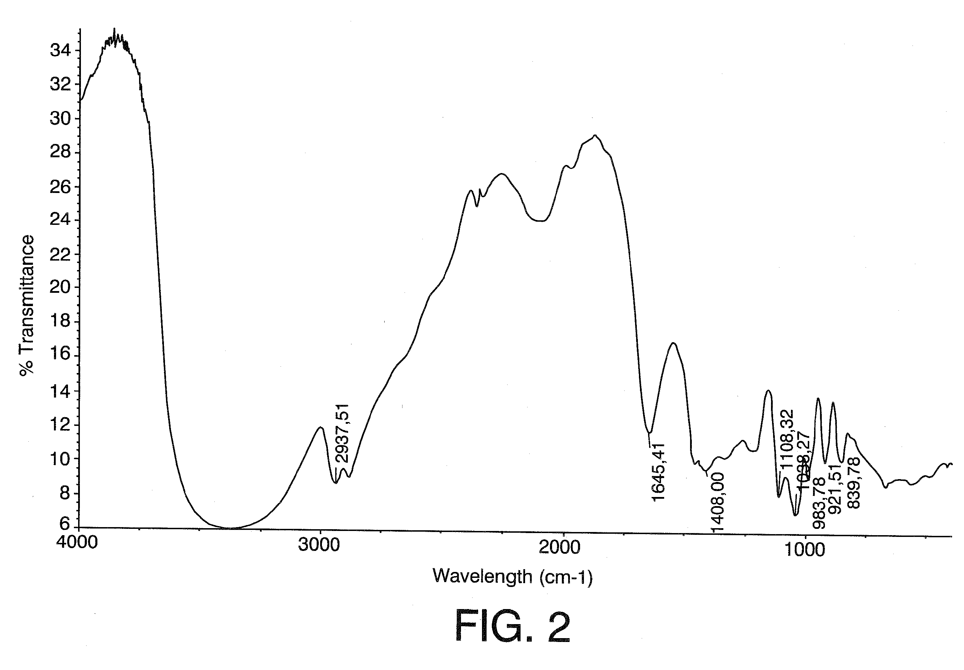

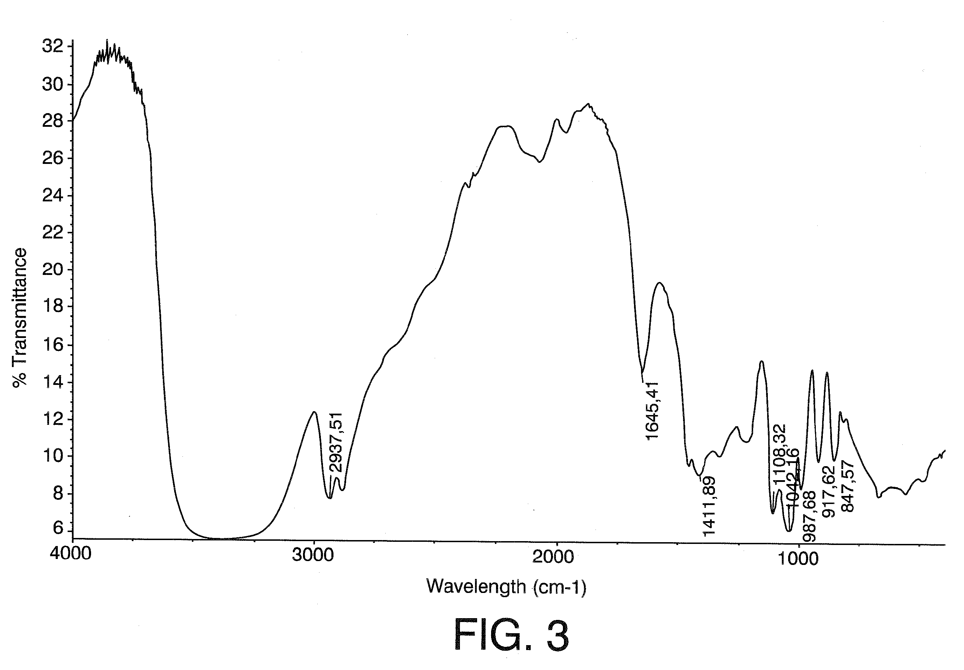

The quality of the glycerin produced was also analyzed by infrared spectroscopy and it was compared with the spectrum from glycerin 85% from the European Directorate for the Quality of Medicines (Figures 2 and 3, respectively).

When comparing the spectra of Figures 2 and 3, it can be observed that the glycerin produced in this work shows the characteristic bands (2937.51 cm⁻¹, 1645.41 cm⁻¹, 1408.00 cm⁻¹, 1108.32 cm⁻¹, 1038.27 cm⁻¹, 983.78 cm⁻¹, 921.51 cm⁻¹ and 839,78 cm⁻¹) of glycerin 85% of the European Directorate for the Quality of Medicines.

In view of this description and set of figures, the person skilled in the art will understand that the embodiments of the invention that have been described can be combined in many ways within the object of the invention.

## Claims

1. Process for purifying glycerin derived from biodiesel production, the process being **characterized in that** it comprises:
a) acidifying the glycerin;
b) treating the acidified glycerin with a base;
c) feeding the glycerin into an electrodialysis unit comprising at least one cation exchange membrane and at least one anion exchange membrane separated between an anode and a cathode, such that they define at least one diluted compartment and at least one concentrated compartment located between the anode and the cathode, wherein glycerin is fed into the diluted compartment and an NaCl solution is fed into the concentrated compartment;
d) applying a potential difference between the anode and the cathode so that the ions present in the glycerin migrate through the anion and cation exchange membranes from the diluted compartments to the concentrated compartments, where they are trapped, purifying the glycerin.
e) extracting the purified glycerin from the electrodialysis unit.

2. Process according to claim 1, **characterized in that** it additionally comprises the step of diluting the neutralized glycerin before being fed into the electrodialysis unit.

3. Process according to claim 2, **characterized in that** the glycerin is diluted with water up to 50%.

4. Process according to any of claims 1 to 3, **characterized in that** it additionally comprises the step of clarifying the glycerin extracted with an adsorbent agent.

5. Process according to claim 4, **characterized in that** the adsorbent agent is selected from the group comprising activated charcoal, clays and ion exchange resins.

6. Process according to any of claims 1 to 5, **characterized in that** it comprises the additional step of removing the water present in the glycerin that has been treated.

7. Process according to claim 6, **characterized in that** the water is removed by means of evaporation.

8. Process according to any of claims 1 to 7, **characterized in that** the applied current density in the electrodialysis unit is 100 to 500 A/m².

9. Process according to any of claims 1 to 8, **characterized in that** the acidification is performed until obtaining a pH of 4 to 5.

10. Process according to any of claims 1 to 9, **characterized in that** the acidification is performed with hydrochloric acid, sulfuric acid or phosphoric acid.

11. Process according to any of claims 1 to 10, **characterized in that** the treatment with the base is performed until obtaining a pH of 6.

12. Process according to any of claims 1 to 11, **characterized in that** the acidification is performed with sodium hydroxide, sodium borohydride, calcium hydroxide, potassium hydroxide or mixtures thereof.

13. Process according to any of claims 1 to 12, **characterized in that** the process is performed in a continuous manner.
